# EUROPEAN PATENT SPECIFICATION

(11) **EP 1 809 260 B1**
(45) Date of publication and mention of the grant of the patent: **27.11.2013**
(21) Application number: 05849078.0
(22) Date of filing: 07.11.2005
(51) Int. Cl.: A61K 9/28, A61K 9/50

(54) **AQUEOUS PHARMACEUTICAL COATING**
WÄSSRIGES PHARMACEUTISCHES COATING
REVETEMENT PHARMACEUTIQUE AQUEUX

(30) Priority: 08.11.2004 IN MU12012004
(43) Date of publication of application: 25.07.2007
(73) Proprietor: Rubicon Research Private Limited, Mumbai 400 078 Maharashtra (IN)
(72) Inventor: PILGAONKAR, Pratibha Sudhir 801/l, Neelam Nagar, Mumbai 400 081 (IN); RUSTOMJEE, Maharukh Tehmasp Dhun Building, Mumbai 400 007 (IN); GANDHI, Anilkumar S. 5b/25, Shree Jawahar Society, Mumbai 400 092 (IN); NAIK, Jyoti Sunil, Mumbai 400 024 (IN)
(74) Representative: O'Neill, Michelle
(86) International application number: PCT/IN2005/000361
(87) International publication number: WO 2006/048895

(56) References cited:
- EP-A- 1 579 771
- WO-A-87/04070
- WO-A-2004/083310
- GB-A- 1 594 102
- US-A- 4 341 563
- US-A- 5 023 108
- US-A- 5 268 181
- US-B1- 6 620 431

## Description

### Field of the Invention

The present invention relates to stable aqueous coating emulsions of wax or combination of waxes.

The present invention also relates to pharmaceutical, food and cosmetic applications of the novel aqueous coating dispersions of wax or combination of waxes.

In particular the pharmaceutical applications include taste masking, stabilization and release retardation of drugs.

### Background of the Invention

Pharmaceutical dosage forms are coated for various reasons including (1) protecting the drug from its surrounding environment (particularly air, moisture, and light) with a view to improving stability, (2) masking of unpleasant taste and odour, (3) increasing the ease by which the product can be ingested by the patient, (4) improving the product identity, (5) facilitating handling, particularly high speed packaging / filling lines, and automated counters in pharmacies, where the coating minimizes cross-contamination due to dust elimination , (6) improving product appearance, particularly where there are noticeable visible differences in the tablet core ingredients from batch to batch, (7) reducing the risk of interaction between incompatible components, (8) improving product mechanical integrity, since coated products generally are more resistant to mishandling (abrasion, attrition, etc), (9) modifying drug release, as in enteric-coated, repeat-action and sustained-release products.

Pharmaceutical dosage forms are coated using various polymeric or nonpolymeric materials dissolved or dispersed in an aqueous or non-aqueous solvent. Use of organic solvents has faced a lot of regulatory restrictions over time due to its inherent cumbersome procedures leading to undesirable effects. These include explosion, fire hazard, toxic release, all of which lead to environmental pollution. Due to the regulatory and manufacturing issues, pharmaceutical companies are rapidly replacing the organic solvents based coating procedures with aqueous film coating processes.

In the past, waxes have been employed for coating, which give better retardation of drug release due to their hydrophobic nature compared to hydrophilic polymers. However their use has been restricted to non-aqueous methods or hot melt techniques. Use of wax in the hot melt technique involves melting of the wax which is then employed in various processes such as granulation, spray coating and extrusion-spheronisation. These methods involve cumbersome procedures and require special equipments. In addition, in case of hot melt-technique, during coating stage, the drug is exposed to high temperature, which is not preferred as it may alter or degrade the drug substance.

In prior art are described applications of coating of pharmaceutical products to achieve taste masking, stabilization and release retardation of drugs. These are described below:

US5151433 discloses coating of drug with a protective polymeric coat. The object of the invention is to protect the labile drug from mechanical stress encountered during compression by using a polymer. The patent describes only polymers as protective agent and waxes as protective agent is not anticipated by this invention.

A multiparticulate controlled release selective serotonin reuptake inhibitor (SSRI) formulation for oral administration is claimed in WO 00/71099A1. The formulation comprises particles of drug coated with rate-controlling polymer, which allows controlled release over a period of not less than about 12 hours. The instant invention makes use of wax for rate controlling instead of polymer claimed in the WO 00/71099A1. The polymeric plasticizer employed in the present invention only helps to produce the uniform film and does not retard the release of the active ingredient.

US6448323 describes a dry film coating composition for use in coating pharmaceutical tablets, nutritional supplements, food, confectionery forms, agricultural seeds, and the like, comprises polyvinyl alcohol, a plasticizer such as polyethylene glycol or glycerin, talc, and preferably a pigment/opacifier and lecithin. This patent also describes a film coating composition, however no wax is employed in the coating composition.

None of the above mentioned prior art applications describe use of waxes as a coating agent more particularly from an aqueous base. Thus there is a long felt need in the pharmaceutical industry to have coating dispersions that are not only aqueous based but also have the advantages provided by using wax as a coating agent.

US2001019725 relates generally to a method of manufacture of controlled release particles containing drug, carrier wax and an excipient by hot melt and mechanical processes. US6238704 relates to a process for preparing a sustained-release formulation by: 1) coating drug containing granules successively with layers of wax, ethyl cellulose containing a plasticizer and a water soluble polymer followed by 2) heating at 50-90°C.

The above two prior art examples utilize heat to melt the waxy components unlike the present invention which utilizes coating with an aqueous wax emulsion.

US5023108 relates to a process for coating of pharmaceutical solids using an aqueous wax emulsion (prepared from a spray dried mixture) containing waxes or lipids alone or in combination in the range of 10 to 30%. PCT application W004083310 describes the preparation and use of a water-wax emulsion, comprising: about 10% to about 50% of a hydrogenated vegetable wax; about 50% to about 90% by weight water; about 1 % to about 25% by weight of a surface-active agent, and about 0.02% to about 2.5% of an acid or base, based on the total weight of the emulsion. Applications described include coating of fibrous cellulosic products to improve moisture resistance.

The above two patents describe use of aqueous emulsions containing wax or waxes for coating. However these emulsions contain 10-30% and 10-50% of waxes in comparison with the present invention, which employs wax contents of up to about 10%.

US6620431 discloses film coatings comprising shellac having a specific acid number along with other water soluble resins and/or plasticizers like HPMC, fatty acids (oleic acid, stearic acid etc), glycerine, propylene glycol, PEG, triacetin, triethyl citrate or glyceryl monostearate to enhance the release profile of the film. Shellac, with pH dependent solubility is used herein for enteric coating and targeting to colon. Shellac is a resin with good film forming properties. This patent however does not describe coating systems based on hydrophobic water insoluble waxes.

W08704070 describes aqueous dispersions of waxes/lipids for pharmaceutical coating comprising a wax/lipid, an emulsifying agent and water. These compositions employ 10-30% of wax/lipid and 0.5-5% of emulsifying agent and tend to have 20-30% solid content. Coating systems with high wax content tend to be viscous and require specific equipments for coating. Moreover emulsions described in this patent may be unstable in liquid form as a result of which it requires additional and expensive step of spray drying.

Further US4341563 and G81594102; disclose coating compositions comprising dispersed particles selected from metal salt of higher fatty acid, higher fatty acid or wax in an aqueous solution of water soluble film base and a surface active agent of defined HLB value. These compositions disclose the addition of hydrophobic material to a solution of polymer like HPMC and surfactant thereby forming a polymeric film base and not a film base of emulsified wax for aqueous coating purposes. This patent describes dispersion of wax particle which can be achieved by routine laboratory practices but higher amount of waxes cannot be incorporated in such a particulate form.

The inventors have found that aqueous wax coating dispersion with 0,5-9% by weight of wax and the use of a plasticizer in the emulsified wax results in a continuous, uniform film formation of the wax, demonstrating excellent coating properties and exhibiting all the desirable qualities of an aqueous base coating medium. The coating dispersion described herein has the advantage of aqueous base as against organic solvents that are commonly employed for coating application involving waxes.

### Objects of the invention:

It is thus an object of the invention to provide aqueous-based wax coating dispersions that overcome problems associated with prior art.

It is another object of the present invention to provide a stable aqueous coating dispersion of waxes for coating solid dosage forms.

It is yet another object of the invention to provide wax coating dispersions that combine the advantages of having an aqueous coating base and wax as coating agent.

Another object of the present invention is to provide wax coating dispersions having emulsified wax in combination with a plasticizer rendering stable and uniform film formation.

Another object of the present invention is to provide aqueous wax coating dispersions that retard the release of the drug from the dosage form.

Yet another object of the present invention is to provide aqueous wax coating dispersions that improve the stability of the active compounds.

Yet another object of the present invention is to provide aqueous wax coating dispersions for masking the bitter taste of active ingredients.

### Summary of the Invention

Thus according to an aspect of the present invention, there is provided a aqueous wax coating emulsion comprising
(a). emulsified wax wherein wax content is at 0.5 to 9 % by weight, and
   an emulsifying agent at 0.1 to 10 % by weight, and
(b). a plasticizer at 5 to 40 % by weight on the weight of the wax; and
(c). water
The wax employed is a single wax or a combination of waxes.

According to second aspect of the present invention, there is provided a method of making the aqueous wax coating emulsion comprising:
(a). heating the wax with emulsifying agent at a temperature above the melting point of the wax.
(b). Adding a part of hot water under stirring to the mixture of step (a)
(c). Cooling to room temperature to get a smooth, stable emulsion; and
(d). adding the solution of plasticizer in the remaining amount of water to the mixture of step ( c) under stirring.

According to yet another aspect of the present invention, there is provided a method of preparing the aqueous wax coating emulsion comprising
(a) heating the wax with emulsifying agent and plasticizer at a temperature above the highest melting component of the blend
(b) Adding the a part of hot water under stirring to the mixture of step (a)
(c) Cooling to room temperature to get a smooth, stable emulsion.
(d) Adding the remaining amount of water to the mixture of step ( c) under stirring.

The active composition of the novel aqueous wax coating dispersions described herein is preferably used in coating solid pharmaceutical formulations including but not limited to powders, tablets, granules, pellets, capsules or other suitable solid dosage form.

### Detailed Description of the Invention

The present invention addresses the need for an aqueous wax coating dispersion which can be used for various purposes like retardation of and pulsatile and extended release, taste masking and stabilization of drug substances. According to the invention, the composition of an aqueous wax coating dispersion typically contains in an aqueous medium;
a) a wax or combination of waxes
b) an emulsifying agent, and
c) a plasticizer

### Composition

The compositions of the present invention are discussed in greater detail with respect to the individual ingredients as hereunder:

### Wax component:

Waxes can be individual or mixtures of esters of monohydroxy alcohols, besides other esters and free fatty acids, free alcohols, higher hydrocarbons and natural or hydrogenated oils. Among the waxes derived from plants are carnauba wax and candelilla wax. The waxy substance secreted by glands on the abdomen of the bee is known commonly as beeswax. Waxes of animal origin include wool wax, or lanolin; spermaceti, and Chinese wax. Mineral waxes include ozocerite and paraffin, both composed of hydrocarbons. Japan wax and bayberry (or myrtle) wax are composed chiefly of fats.

Waxes form excellent barrier coatings for moisture sensitive drugs. The system protects the drug molecules from atmospheric moisture without affecting the active ingredient thus providing good stability and protection for hygroscopic drugs. Waxes are also used as retarding agents for drug release.

The preferred wax according to the present invention is selected from carnauba wax, candelilla wax, spermaceti, bees wax, montan wax, microcrystalline wax, lecithin, hydrogenated tallow, paraffin wax, cetyl alcohol, cetostearyl alcohol, stearic acid hydrogenated vegetable oil such as hydrogenated cottonseed oil (lubritab^{™}), hydrogenated soyabean oil (sterotex HMNF ^{™}), compritol, precirol, shellac wax, petrolatum, and the like as well as synthetic waxes, e.g., polyethylene, and the like. These waxes can be used alone or in combinations.

The percentage wax according to present invention is 0.5% - 9% by weight.

The wax is preferably one or more of hydrogenated vegetable oils.

The most preferred percentage wax according to present invention is 2.5-7.5% by weight

### Emulsifying agent:

Emulsifying agent or Emulsifiers generally classified as surfactants and have hydrophilic and lipophilic structural portions within their molecular structures. Pharmaceutically acceptable emulsifiers should also be stable, compatible with other ingredients and non-toxic. It should possess little odor, taste or color and should not interfere with the stability of efficacy of the active agent. The emulsifier may be selected from hydrophilic surfactants or lipophilic surfactants or mixtures thereof. The surfactants may be anionic, nonionic, cationic, and zwitterionic surfactants.

The preferred emulsifying agents according to the present invention are selected from non-ionic emulsifiers like mono- and diglycerides, medium chain glyceride (Capmul) glyceryl monooleate (Peceol), glyceryl ricinoleate, glyceryl laurate and glyceryl caprylate (Capmul MCM), PEG sorbitan fatty acid esters like PEG -20 sorbitan monolaurate (Tween 20), PEG 20 sorbitan monostearate (Tween 60) and PEG sorbitan monooleate (Tween 80), Sorbitan fatty acid esters like sorbitan monolaurate (span 20), sugar ester surfactants like sucrose distearate (sucro ester 7), ionic emulsifiers like sodium caprylate sodium lauryl sulphate, phospholipids, alginate salts. These emulsifiers can be used alone or in combinations.

The emulsifying agent is employed at a percentage of about 0.1-10% by weight. A preferred emulsifying agent is a medium chain glyceride.

The preferred percentage of emulsifying agent according to present invention is about 0.5-7.5% by weight

The most preferred percentage of emulsifying agent according to present invention is 0.75-5.0%, by weight of the total composition.

### Plasticizer:

Plasticizers are used to improve the processibility, flexibility, and elasticity of coating agents. These plasticizing compounds also reduce the brittleness, , and improve the toughness and tensile strength of the coating agents. They play an important role in the film formation process. The quality of the resulting film is dependent on the type and amount of plasticizer added to the coating medium. According to the present invention, plasticizers may be polymeric or nonpolymeric and soluble or insoluble in water.

The preferred plasticizer according to the present invention is selected from polymers like ammonio methacrylate co-polymer, Eudragit RL, Eudragit RS, polyvinyl alcohol, polyvinylpyrrolidone, polyvinyl acetate, mixture of polyvinyl acetate with povidone (Kollidone SR™) methylcellulose, ethyl cellulose, sodium carboxy methylcellulose, hydroxypropylcellulose, hydroxypropylmethyl cellulose or polyethylene glycol, cellulose acetate cellulose propionate (lower, medium or higher molecular weight), cellulose acetate propionate, cellulose acetate butyrate, cellulose acetate phthalate, cellulose triacetate, poly (methyl methacrylate), poly (ethyl methacrylate), poly (butyl methacrylate), poly (isobutyl methacrylate), and poly (hexyl methacrylate), poly (isodecyl methacrylate), poly (lauryl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene) high density, poly (ethylene oxide), poly (ethylene terphthalate) poly (vinyl isobutyl ether), poly (vinyl acetate), poly (vinyl chloride) or polyurethane, polymers based on acrylate and/or methacrylates).

The preferred plasticizer according to the present invention may also be selected from nonpolymeric plasticizers like propylene glycol, glycerine, triethyl citrate, dibutyl sebacate, gelucire 39/01, gelucire 43/01 or any of the nonpolymeric plasticizer with similar plasticizing function.

A preferred plasticizer is selected from polyvinyl alcohol, hydroxypropylcellulose and hydroxypropylmethyl cellulose.

The chosen plasticizer can be used individually or on combinations.

The plasticizer is employed at a concentration of 5 to 40% by weight of the wax, preferably 7.5% to 35% by weight of the wax, and more preferably 10%-30% by weight of the wax.

### Process

The procedures for preparation of aqueous wax coating emulsion containing wither a water soluble or water insoluble plasticizer are described below:
The process for a water soluble plasticizer comprises steps of:
a) heating the wax with emulsifying agent at a temperature above the melting point of the wax.
b) Adding a part of hot water under stirring to the mixture of step (i)
c) Cooling to room temperature to get a smooth, stable emulsion
d) adding the solution of plasticizer in the remaining amount of water to the mixture of step (iii) under stirring.
The process for a water insoluble plasticizer comprises steps of:
a) heating the wax with emulsifying agent and plasticizer at a temperature above the highest melting component of the blend
b) Adding the a part of hot water under stirring to the mixture of step (i)
c) Cooling to room temperature to get a smooth, stable emulsion.
d) Adding the remaining amount of water to the mixture of step (iii) under stirring.

The dispersion obtained from any of the above process can be screened through a 150 micron (100#) sieve in order to confirm the absence of any insoluble material and an uniformity of the dispersion.

### Applications

According to the present invention the aqueous wax coating emulsion can be employed as a coating agent that renders a continuous and uniform film formulation and to apply a wax coat to any suitable solid pharmaceutical form, e.g. drug particles, granules, pellets, tablets, capsules, patches, films. The wax-coated particulates may be compressed or suitably processed into a dosage form. The aqueous wax coating emulsion may be used for controlled release formulations.

Wax coating, according to the current invention, is aimed at achieving stabilization, taste masking and modification of release of a drug substance. One skilled in the art will also appreciate that the coating dispersion described herein can replace any polymer coating known thus far in the field of pharmaceutical coating. Stabilization of drug substance can be in respect of hydrolytic, oxidative or any other form of degradation. Modified drug release can be in the form of sustained, pulsatile, delayed, or targeted release.

The details of the invention, its objects and advantages are explained hereunder in relation to non-limiting exemplary illustrations.

### Example 1.

### Preparation of aqueous wax coating dispersion using hydrogenated vegetable oil and a water soluble plasticizer

**Table 1. Composition for the preparation of aqueous wax coating dispersion**

| **Ingredient** | **%w/w** |
|---|---|
| Hydrogenated Vegetable oil Type 1 | 7.50 |
| (Lubritab) | |
| Medium chain glycerides | 2.00 |
| (Capmul MCM) | |
| Polyvinyl alcohol | 2.25 |
| (PVA18-88) | |
| Demineralised Water | q.s. |

Lubritab and Capmul MCM were heated in a water bath at 80°C -85°C. A part of the water was heated to 90°C and added to the molten waxy mass with constant stirring using a suitable laboratory stirrer to obtain a smooth emulsion. The emulsion was cooled to room temperature. The plasticizer was dissolved in the remaining water, and added to the above emulsion under stirring.
A stable and uniform dispersion was obtained.

### Example 2.

### Preparation of aqueous wax coating dispersion using hydrogenated soyabean oil and a water soluble plasticizer

**Table 2. Composition for the preparation of aqueous wax coating dispersion**

| **Ingredient** | **%w/w** |
|---|---|
| Hydrogenated soyabean oil (Sterotex HMNF) | 7.50 |
| Medium chain glycerides (Capmul MCM) | 2.00 |
| Hydroxypropyl methylcellulose (Methocel E-5) | 2.25 |
| Demineralised Water | q.s. |

Sterotex and Capmul MCM were heated in a water bath at 70°C -80°C. A part of the water was heated to 90°C and added to the molten waxy mass with constant stirring using a suitable laboratory stirrer to obtain a smooth emulsion. The emulsion was cooled to room temperature. The plasticizer was dissolved in the remaining quantity of water and added to the above emulsion under stirring.
A stable and uniform dispersion was obtained.

### Example 3

### Stability of aqueous wax coating dispersion

The aqueous wax coating dispersion prepared in Example 1 and Example 2 were observed visually for physical stability such as cracking (separation of phases) or creaming of oil droplets at the surface after 1, 7, 15 and 30 days storage at the ambient.

It was observed that both the compositions were stable at the end of 30 days as shown by absence of creaming and cracking.

### Example 4.

### Preparation of aqueous wax coating dispersion using water insoluble plasticizer

**Table 3. Composition for the preparation of aqueous wax coating dispersion**

| **Ingredient** | **%w/w** |
|---|---|
| Hydrogenated Vegetable oil Type 1 | 7.50 |
| (Lubritab) | |
| Medium chain glycerides | 2.00 |
| (Capmul MCM) | |
| Glyceryl ester of saturated fatty acids (Gelucire 39/01) | 1.5 |
| Demineralised Water | q.s. |

Lubritab, Gelucire 39/01 and Capmul MCM were heated in a water bath at 80°C -85°C. A part of the water was heated to 90°C and added to the molten waxy mass with constant stirring using a suitable laboratory stirrer to obtain a smooth emulsion. The emulsion was cooled to room temperature. The remaining quantity of water was added to the above emulsion under stirring.
A uniform dispersion was obtained which was observed to be stable on storage.

### Example 5.

### Comparative example as per US Patent No. 5023108.

**Table 4. Composition of wax dispersion**

| **Ingredient** | **%w/w** |
|---|---|
| Hydrogenated cotton seed oil | 25.00 |
| Polyoxyethylene 20 sorbitan monooleate | 1.50 |
| Veegum | 1.00 |
| Distilled Water | q.s. |

Hydrogenated cottonseed oil was heated in a water bath at 80°C-85°C. A 10% solution of Polyoxyethylene 20 sorbitan monooleate heated to 90° C, was then added to the molten lipid mass with constant stirring using a suitable laboratory mixer. A 4% dispersion of Veegum in water was heated to 90°C and added with stirring to above dispersion. The crude emulsion thus formed was then homogenized and shock-cooled in an ice-bath with constant stirring, a solid mass (gritty particles) was formed.

This observation demonstrates the inadequacy of this prior art system and the superiority of the compositions of the present invention through the use of a plasticizer and lower levels of wax content.

### Example 6

### Preparation of aqueous wax coating dispersion without plasticizer to demonstrate the effect on film characteristics

**Table 5. Composition for the preparation of aqueous wax coating dispersion**

| Ingredient | %w/w |
|---|---|
| Hydrogenated vegetable oil Type 1 (Lubritab) | 7.5 |
| Medium chain glycerides (Capmul MCM) | 2.0 |
| Distilled Water | q.s. |

Hydrogenated vegetable oil and Capmul MCM were heated in a water bath at 80°C -85°C. A part of the water was heated to 90°C and added to the molten waxy mass with constant stirring using a suitable laboratory stirrer to obtain a smooth emulsion. The emulsion diluted with remaining quantity of water and cooled to room temperature.

Films were cast using above emulsion and dispersion of Example 1 and dried at 40°C for 6-8 hours.

Films obtained with dispersion of Example I were smooth and had minimum cracks whereas films of above dispersion gave discontinuous, cracked appearance. This data indicates the need of using a plasticizer in the aqueous wax coating dispersion. Plasticizer helps to reduce the brittleness of the film and improves the tensile strength of the film which is essential for a coating dispersion.

### Example 7.

### Preparation of aqueous wax coating dispersion using glycerin as a nonpolymeric plasticizer

**Table 6. Composition for the preparation of aqueous wax coating dispersion**

| **Ingredient** | **%w/w** |
|---|---|
| Hydrogenated Vegetable oil Type 1 | 7.50 |
| (Lubritab) | |
| Medium chain glycerides (Capmul MCM) | 2.00 |
| Glycerin | 1.50 |
| Demineralised Water | q.s. |

Hydrogenated vegetable oil and Capmul MCM were heated in a water bath at 80°C -85°C. A part of the water was heated to 90°C and added to the molten waxy mass with constant stirring using a suitable laboratory stirrer to obtain a smooth emulsion. The emulsion was cooled to room temperature. In the remaining quantity of water, the plasticizer was dissolved and added to the above emulsion under stirring.

A uniform dispersion was obtained, which showed desirable stability on storage.

### Example 8.

### Preparation of aqueous wax coating dispersion using a combination of hydroxypropyl methylcellulose and polyethylene glycol as plasticizer

**Table 7. Composition for the preparation of aqueous wax coating dispersion**

| **Ingredient** | **%w/w** |
|---|---|
| Hydrogenated Vegetable oil Type 1 | 7.50 |
| (Lubritab) | |
| Medium chain glycerides (Capmul MCM) | 4.00 |
| Hydroxypropyl methylcellulose (Methocel E-5) | 2.25 |
| Polyethylene glycol 8000 | 2.25 |
| Demineralised Water | q.s. |

Hydrogenated vegetable oil and Capmul MCM were heated in a water bath at 80°C -85°C. A part of the water was heated to 90°C and added to the molten waxy mass with constant stirring using a suitable laboratory stirrer to obtain a smooth emulsion. The emulsion was cooled to room temperature. In the remaining quantity of water, the plasticizers were dissolved and added to the above emulsion under stirring.
A uniform dispersion was obtained which was stable on storage.

### Example 9.

### Preparation of aqueous wax coating dispersion using aqueous dispersions of polymers as plasticizer

**Table 8. Composition for the preparation of aqueous wax coating dispersion**

| **Ingredient** | **%w/w** | **%w/w** |
|---|---|---|
| Hydrogenated Vegetable oil Type 1 | 7.50 | 7.50 |
| (Lubritab) | | |
| Medium chain glycerides (Capmul MCM) | 1.00 | 1.00 |
| Ethyl cellulose (Surelease aqueous dispersion) | 2.25 | - |
| Ammonia methacrylates copolymer dispersion (Eudragit RS 30D) | - | 2.25 |
| Triethyl citrate | - | 0.225 |
| Demineralised Water | q.s. | q.s. |

Hydrogenated vegetable oil and Capmul MCM were heated in a water bath at 80°C -85°C. A part of the water was heated to 90°C and added to the molten waxy mass with constant stirring using a suitable lab stirrer to obtain a smooth emulsion. The emulsion was cooled to room temperature. In the remaining quantity of water, plasticizers in the form of aqueous dispersions were mixed and added to the above emulsion under stirring.
Uniform dispersions were obtained which showed good stability on storage.

### Example 10

### Preparation of aqueous wax coating dispersion using cetostearyl alcohol

**Table 9. Composition for the preparation of aqueous wax coating dispersion**

| **Ingredient** | **%w/w** |
|---|---|
| Cetostearyl alcohol | 5.00 |
| Medium chain glycerides (Capmul MCM) | 4.00 |
| Hydroxypropyl methylcellulose (Methocel E-5) | 2.25 |
| Demineralised Water | q.s. |

Cetostearyl alcohol and Capmul MCM were heated in a water bath at 80°C -85°C. A part of the water was heated to 90°C and added to the molten waxy mass with constant stirring using a suitable lab stirrer to obtain a smooth emulsion. The emulsion was cooled to room temperature. In the remaining quantity of water, plasticizer was dissolved and added to the above emulsion under stirring.
A uniform stable dispersion was obtained.

### Example 11

### Preparation of aqueous wax coating dispersion using compritol

**Table 10. Composition for the preparation of aqueous wax coating dispersion**

| **Ingredient** | **%w/w** |
|---|---|
| Glyceryl behenate (Compritol ATO 888) | 5.00 |
| Medium chain glycerides (Capmul MCM) | 4.00 |
| Hydroxypropyl methylcellulose (Methocel E-5) | 2.25 |
| Demineralised Water | q.s. |

Compritol and Capmul MCM were heated in a water bath at 80°C -85°C. A part of the water was heated to 90°C and added to the molten waxy mass with constant stirring using a suitable lab stirrer to obtain a smooth emulsion. The emulsion was cooled to room temperature. In the remaining quantity of water, plasticizer was dissolved and added to the above emulsion under stirring.
A uniform stable dispersion was obtained.

### Example 12

### Preparation of aqueous wax coating dispersion using Tween 80 as emulsifier

**Table 11. Composition for the preparation of aqueous wax coating dispersion**

| **Ingredient** | **%w/w** |
|---|---|
| Hydrogenated Vegetable oil Type 1 | 7.50 |
| (Lubritab) | |
| Tween 80 | 4.00 |
| Hydroxypropyl methylcellulose (Methocel E-5) | 2.25 |
| Demineralised Water | q.s. |

Hydrogenated Vegetable oil and Tween 80 were heated in a water bath at 80°C -85°C. A part of the water was heated to 90°C and added to the molten waxy mass with constant stirring using a suitable lab stirrer to obtain a smooth emulsion. The emulsion was cooled to room temperature. In the remaining quantity of water, plasticizer was dissolved and added to the above emulsion under stirring.
A uniform stable dispersion was obtained.

### Example 13

### Effect of wax content on the quality of aqueous wax coating dispersion.

**Table 12: Composition for the preparation of aqueous wax coating dispersion**

| **Ingredient** | **%w/w** | | | |
|---|---|---|---|---|
| | A | B | C | D |
| Hydrogenated soyabean oil (Sterotex HMNF) | 9.50 | 12.0 | 12.0 | 12.0 |
| Medium chain glycerides (Capmul MCM) | 10.00 | 10.0 | 10.0 | 10.0 |
| Hydroxypropyl methylcellulose (Methocel E-5) | 3.8 | 4.8 | 3.6 | 1.8 |
| Demineralised Water | q.s. | q.s. | q.s. | q.s. |

Sterotex and Capmul MCM were heated in a water bath at 70°C -80°C. A part of the water was heated to 90°C and added to the molten waxy mass with constant stirring using a suitable laboratory stirrer to obtain a smooth emulsion. The emulsion was cooled to room temperature. In the remaining quantity of water, the plasticizer was dissolved and added to the above emulsion under stirring. In this example following variables were attempted:
Composition A: Wax 9.5% w/w and plasticizer - 40% by weight of wax, which is as per the present invention
Composition B: Wax 12% w/w and plasticizer - 40% by weight of wax
Composition C: Wax 12% w/w and plasticizer - 30% by weight of wax
Composition D: Wax 12% w/w and plasticizer - 15% by weight of wax

It was observed that at 9.5% w/w of wax a smooth and uniform, sprayable dispersion was obtained, however at 12% w/w of wax, the emulsion was very thick, had gritty appearance which can not be sprayed for coating purposes. Even with plasticizer concentration of 15% by weight of wax (Example D), the dispersion was not sprayable.

This clearly demonstrates that above 10%w/w concentration of wax, the dispersion loses its liquid consistency and cannot be used as a coating agent.

### Example 14

### Use of aqueous wax coating dispersion to obtain pulsed and sustained release of a highly water-soluble drug.

Drug used: Metformin Hydrochloride
Dosage form: Tablets

### (a) Preparation of Tablets for coating:

**Table 13 Composition of core tablets**

| **Ingredient** | **mg per tablet** |
|---|---|
| Metformin Hydrochloride | 500.00 |
| Povidone (PVP K-90) | 37.50 |
| Magnesium stearate | 5.00 |

Metformin hydrochloride and PVP K-30 weighed and sifted through mesh 40 and loaded in a Planetary Mixer and mixed for 10 minutes and then granulated using demineralized water. The wet mass was then dried in fluidized bed dryer at inlet air temperature of 60°C and bed temperature at 45°C. The dried granules were then passed through mesh 16 and lubricated with magnesium stearate and compressed using capsule shaped punches.

### (b) Coating of Tablets using aqueous wax coating dispersion of example 1:

800 g of Metformin tablets were placed in the coating equipment. The tablets were sprayed with the coating dispersion of Example 1 employing methods known in the art. The inlet temperature was kept at 60° C. The spraying was carried out continuously until the desired increase in mean weight of tablets was achieved. Tablets thus obtained had a smooth and lustrous surface.

**Table 14. Dissolution profile of coated Metformin tablets**

| Time (hrs) | % Released | | | |
|---|---|---|---|---|
| | Uncoated | 5%coated | 13%coated | 18%coated |
| 1hr | 102.83 | 98.21 | 28.30 | 5.30 |
| 2hr | - | - | 95.79 | 80.50 |
| 4hr | - | - | 101.36 | 97.91 |

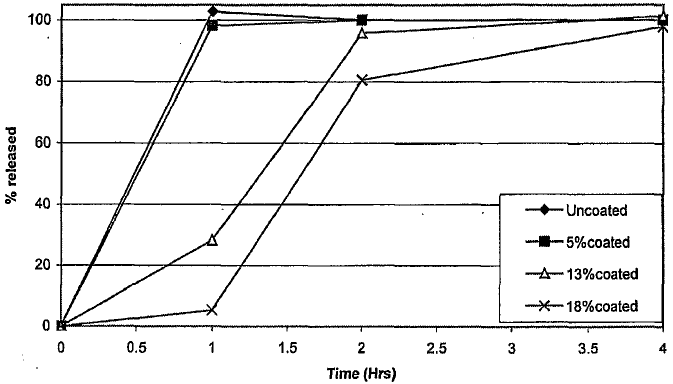

Dissolution profiles indicate that at a coating of 18%, a lagtime of about one hour in the release of metformin was observed and in the next 1 hour about 80% of the drug was released. This suggests that aqueous wax coating can be employed to develop pulsed delivery systems with a defined lag period.

### (c) Coating of Tablets using aqueous wax coating dispersion of example 2:

200 g of Metformin tablets were placed in the coating vessel of fluidized bed processor. The tablets were sprayed with the coating emulsion of Example 2 according to the methods known in the art. The inlet temperature was kept at 60°C. The spraying was carried out continuously until the desired increase in mean weight of tablets was achieved. Tablets thus obtained had a smooth and lustrous surface.

**Table 15. Dissolution profile of coated Metformin tablets**

| Observation | | | |
|---|---|---|---|
| Time (Hrs) | % Release | | |
| | Uncoated | 13%coated | 18%coated |
| 1hr | 102.83 | 0.04 | 0.60 |
| 2hr | - | 4.60 | 4.60 |
| 4hr | - | 61.20 | 43.30 |
| 6hr | - | 89.40 | 87.00 |
| 8hr | - | 93.90 | 99.2 |

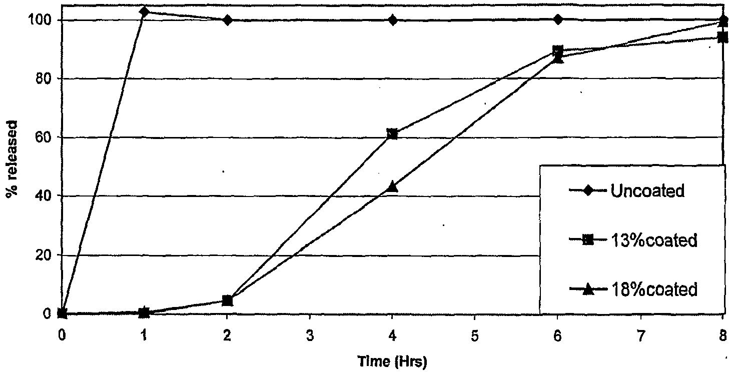

At both the coating levels after a lag period of 2 hour a sustained release of drug was obtained over 8 hours.

These results indicate that pulsed or sustained release of a highly water soluble drug such as metformin can be achieved by selecting appropriate coating dispersion composition and coating level.

### Example 15

### Use of aqueous wax coating dispersion to retard release of metoprolol succinate

### (a) Preparation of Metoprolol succinate pellets

**Table 16 Composition of core pellets**

| **Ingredients** | **%w/w** |
|---|---|
| Metoprolol succinate | 80.00 |
| Microcrystalline cellulose (Pharmacel 101) | 20.00 |
| Povidone(PVP K-30) | 1.00 |

Drug and Pharmacel 101 were sifted together through mesh 40 (425 micron) and mixed in low shear mixer. Povidone was dissolved in the required quantity of water. This solution was added to the above mixture under mixing till a wet mass of proper consistency was obtained. The wet mass was then extruded through an extrudor using a 0.5mm size cone plate attachment and spheronised. The pellets were dried at 50°C for 10 min

### (b) Preparation of aqueous wax coating dispersion

**Table 17 Composition of aqueous wax coating dispersion**

| **Ingredient** | **%w/w** |
|---|---|
| Hydrogenated Vegetable oil Type 1 (Lubritab) | 7.50 |
| Medium chain glyceride (Capmul MCM) | 2.00 |
| Hydroxy propyl cellulose (Klucel LF) | 1.5 |
| Demineralised Water | q.s. |

Hydrogenated vegetable oil and Capmul MCM were heated in a water bath at 80°C -85°C. A part of the water was heated to 90°C and added to the molten waxy mass with constant stirring using a suitable lab stirrer to obtain a smooth emulsion. The emulsion was cooled to room temperature. In the remaining quantity of water, plasticizer was dissolved and added to the above emulsion under stirring.

### (c) Coating of Metoprolol succinate pellets:

80g of pellets (#20 to #60) were placed in the coating vessel of fluidized bed processor. The pellets were sprayed with the above dispersion according to the method known in the art. The inlet temperature was kept at 50°C. The spraying was carried out continuously until the desired coating level was achieved. Pellets thus obtained had a smooth and lustrous surface. Coated beads were then filled into capsules and dissolution was carried out in pH 6.8 phosphate buffer.

**Table 18. Dissolution profile of coated beads**

| Time(hr) | % Release(15% coated) |
|---|---|
| 0 | 0.0 |
| 1 | 65.3 |
| 2 | 77.1 |

These data suggest that using aqueous wax coating dispersion of present invention, it is possible to coat pellets and retard release of a highly water soluble drug such as metoprolol.

### Example 16

### Use of aqueous wax coating dispersion to retard release of diclofenac sodium

### (a). Preparation of diclofenac tablets

**Table 19 Composition of core tablets**

| **Ingredients** | **Mg/tab** |
|---|---|
| Diclofenac sodium | 75.00 |
| Povidone (PVPK-30) | 2.00 |
| Lactose monohydrate | 26.00 |
| Starch | 22.00 |
| Microcrystalline cellulose (Avicel pH 102) | 15.00 |
| Magnesium stearate | 1.00 |

Starch, Lactose and Diclofienac sodium were granulated using a solution of polyvinyl pyrrolidone in purified water and dried in fluidized bed dryer till desired moisture content is achieved. Granules were then blended with other excipients, lubricated and compressed into tablets using 7.0mm standard concave punches.

### (b) Coating of tablets:

Tablets were coated in a conventional coating pan using aqueous wax coating dispersion containing Sterotex HMNF (Refer composition Example 2). Samples of coated tablets were collected at 5%, 10%, 15% and 20% weight gain levels and release of active agent was determined using pH 6.8 phosphate buffer as dissolution medium.

**Table 20. Dissolution profile of coated tablets**

| Time (min) | % Release | |
|---|---|---|
| | 15%coated | 20%coated |
| 0 | 0 | 0 |
| 10 | 10.8 | 11.2 |
| 20 | 11.4 | 11.8 |
| 30 | 24.8 | 11.8 |
| 45 | 46.4 | 20.5 |
| 60 | 73.5 | 47.1 |
| 90 | 88.5 | 69.3 |
| 120 | 93.9 | 83.8 |
| 240 | 97.8 | 88.7 |

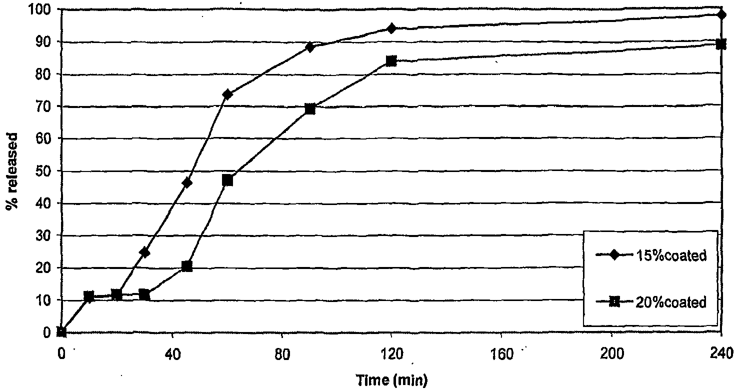

The results suggest that controlled release of a drug having intermediate solubility in water can be obtained using aqueous wax coating dispersions of present invention.

### Example 17

### Use of aqueous wax coating dispersion for stabilization of Tibolone

### (a) Preparation of tibolone pellets:

**Table 21 Composition of tibolone pellets:**

| **Ingredients** | **%w/w** |
|---|---|
| Tibolone | 10.00 |
| Microcrystalline cellulose (Pharmacel 101) | 25.00 |
| Povidone (PVP K-30) | 3.00 |
| Lactose Monohydrate (Pharmatose 200M) | 62.0 |

Drug, Lactose and microcrystalline cellulose were sifted together through mesh 40 (425 micron) and mixed in low shear mixer. Povidone was dissolved in the required quantity of water and added to the above mixture under mixing till a wet mass of proper consistency obtained. The wet mass was then extruded through an extrudor using a 0.5mm size cone plate attachment and spheronised. The pellets were dried at 40°C.

### (b) Preparation of Coating dispersions

**Table 22 Composition of coating dispersions:**

| **Ingredient** | **%w/w** |
|---|---|
| **(a) Aqueous wax coating dispersion** | |
| Hydrogenated Vegetable oil Type 1 (Lubritab) | 7.50 |
| Medium chain glyceride (Capmul MCM) | 2.00 |
| Hydroxy propyl methyl cellulose (Methocel E-5) | 0.75 |
| Dematerialized Water | q.s. |

| **(b) HPMC coating solution** | |
|---|---|
| Hydroxy propyl methyl cellulose (Methocel E-3) | 10.00 |
| Polyethylene Glycol 8000 | 3.00 |
| Talc | 8.00% |
| Demineralized Water | q.s. |

| | |
|---|---|
| (a) Process for preparing aqueous wax coating dispersion was same as given in example 2 (b) In the half of the amount of water, PEG 8000 was dissolved and talc was dispersed in it. HPMC was dissolved in the remaining amount of water and added to above dispersion. The suspension was filtered through #100 mesh (150 micron)screen | |

### (c) Coating of Tibolon pellets:

Pellets (#20 to #60) were placed in the coating vessel of fluidized bed processor. The pellets were sprayed with the above coating dispersion (either a or b) according to the method known in the art. The inlet temperature was kept at 45°C. The spraying was carried out continuously until the desired coating level was achieved. Pellets thus obtained had a smooth and lustrous surface. Coated beads were then exposed at accelerated conditions of 40°C/38% RH and 40°C/75% RH for 15 days. The levels of impurity C of tibolon were determined using HPLC for the uncoated, HPMC coated and aqueous wax dispersion coated beads.

**Table 23: Levels of impurity c of tibolone**

| Composition | Impurity C (%) 40°C/38% RH | Impurity C (%) 40°C/75% RH |
|---|---|---|
| Uncoated beads | 0.10 | 0.21 |
| Aqueous wax dispersion coated beads | 0.06 | 0.17 |
| HPMC coated beads | 0.10 | 0.33 |

Under both the accelerated conditions, it was found that the amount of impurity C was lowest for beads coated with the aqueous wax dispersion, suggesting that the aqueous wax coating dispersion can be employed to stabilize actives sensitive to temperature and/or moisture.

### Example 18

### Use of aqueous wax coating dispersion to stabilize ascorbic acid (Vitamin C)

### (a) Preparation of ascorbic acid tablets

Vitamin C was granulated using molten Compritol. The mass was cooled to room temperature and further blended with Kollidone SR, Kollidone VA 64 and magnesium stearate. The blend was compressed using capsule shaped punches.

### (b) Coating of tablets:

The compressed tablets were coated in a conventional coating pan with wax coating dispersion of example 2 till a coating level of 5% was achieved.

Both coated and uncoated tablets were placed in an open petriplate at 40°C/75% RH. After a week it was observed that the color of the uncoated tablets had changed to dark yellow both on the surface as well as inside the tablet (observed by cutting tablets into two halves) indicating degradation of ascorbic acid. However, the coated tablets were only pale yellow in color when tablets were cut into two halves. This example also suggest that aqueous wax coating dispersion can be employed to stabilize actives which may be susceptible to degradation either by oxidation, heat or humidity or a combination of these.

### Example 19

### Use of aqueous wax coating dispersion to stabilize phenytoin sodium

Phenytoin sodium shows incompatibility and discoloration when physically mixed with lactose in pharmaceutical compositions. Wax coating approach was applied to stabilize the phenytoin in presence of lactose.

Plain phenytoin sodium was sifted through 40 # and mixed with MCC (Avicel PH 102) [1:1 ratio] and the mixture was loaded in fluidized bed processor. Coating of this mixture was carried out using wax coating dispersion of example 2.

The phenytoin sodium-MCC mixture with a wax coating level of 10 %, was used for the physical incompatibility study by mixing with lactose (pharmatose 200 M) in a 1:2 ratio. The mixture was sifted through 40# and the samples were subjected to an accelerated stability study at room temperature and 40 degree/75% RH. A blend of uncoated phenytoin sodium-MCC mixture with Lactose (pharmatose 200) in the 1:2 was used as positive control. Samples were observed for physical changes in appearance and color.

After an exposure for 3 days the color of the uncoated phenytoin blend had changed to yellow whereas coated phenytoin remained only slightly colored. Thus aqueous wax coating dispersion can be employed to prevent drug excipient interaction and thereby stabilize the drug.

### Example 20

### Use of aqueous wax coating dispersion to mask the taste of drug

Drug used: Ursodiol
Dosage form : Dispersible Tablets

### (a) Preparation of aqueous wax coating dispersion

**Table 24 Composition of aqueous wax coating dispersion**

| Ingredient | %w/w |
|---|---|
| Hydrogenated vegetable oil Type 1 (Lubritab) | 10.0 |
| Medium chain glycerides (Capmul MCM) | 2.0 |
| Polyvinyl alcohol (PVA18-88) | 3.0 |
| Distilled Water | q.s. |

The coating dispersion was prepared as described in Example 1. This wax emulsion was used as a binder in the preparation of Ursodiol tablets.

### (b) Preparation of Tablets

**Table 25 Composition of ursodiol tablets**

| Ingredient | Mg/tablet |
|---|---|
| Ursodiol | 75.0 |
| Mannitol | 75.0 |
| Crosscamellose sodium | 18.0 |
| Crospovidone | 12.0 |
| Talc | 6.0 |
| Colloidal silicon dioxide | 3.0 |
| Aspartame | 35.0 |
| Taste Masking flavor | 0.9 |
| Strawberry flavor | 2.1 |
| Magnesium stearate | 3.0 |

Ursodiol was granulated with the wax emulsion and the granules thus obtained were mixed with mannitol, passed through mesh 8 and then dried in the tray dryer at 45°C. The dried granules were then passed through mesh 20 and mixed with the remaining excipients and compressed into tablets.

### (c) Evaluation of tablets with respect to taste in human volunteers

A panel of 10 human volunteers was selected. These tablets were dispersed in 15 ml of water and this dispersion was given to all the volunteers. The bitter taste of ursodiol was masked and the tablets were acceptable with respect to overall mouthfeel, taste and palatability.

Thus, granulation with aqueous wax dispersion can be employed for masking the bitter taste of drug such as ursodiol and this drug subsequently incorporated into dispersible tablet formulations.

### Example 21

### Use of aqueous wax coating dispersion for masking the bitter taste of cetirizine hydrochloride

### (a) Preparation of powder blend of cetirizine hydrochloride with F-melt

**Table 26 Blend of cetirizine hydrochloride with F-melt**

| Ingredients | mg/unit |
|---|---|
| Cetirizine Hydrochloride | 1.00 |
| F-melt™ | 2.00 |

Accurately weighed amounts of cetirizine hydrochloride and F-melt were passed through 40# and mixed to obtain uniform blend.

### (b) Coating of blend of Cetirizine Hydrochloride-F-melt

The blend of Cetirizine hydrochloride-F-melt was placed in the coating vessel of fluidized bed processor. The blend was sprayed with the coating emulsion of example 16 employing methods known in the art. The inlet temperature was kept at 60°C. The spraying was carried out continuously until the desired coating level of 6% was achieved.

### (c) Preparation of mouth dissolve tablets

**Table 27 Composition of mouth dissolve tablets**

| Ingredients | mg/tab |
|---|---|
| Wax coated Cetirizine Hydrochloride | 15 |
| F-melt | 49 |
| Xylisorb | 15.75 |
| Polyplasdone XL 10 | 6 |
| Sodium saccharine | 2 |
| Strawberry flavour | 0.25 |
| Colorcon taste mask flavour TM | 1 |
| Magnesium stearate | 1 |
| Total | 90 mg |

F-melt, xylisorb and polyplasdone were weighed, sifted through 40 # and mixed uniformly. The above blend was then mixed with wax coated drug for 10 mins. Weighed amounts of sweeteners and flavors were sifted through 60# and mixed with above blend to obtain uniform mixture. The entire blend was then passed through 40 # and remixed. The above blend was then lubricated with magnesium stearate and compressed to tablets using capsule shaped punches on a compression machine.

The above process was also followed for plain uncoated cetirizine hydrochloride as control samples.

### (d) Evaluation of tablets with respect to taste and mouth feel in human volunteers

A panel of 10 human volunteers was selected and all the volunteers were given one tablet each of both the samples i.e. wax coated cetirizine hydrochloride tablets and uncoated tablets. These tablets were kept on the tongue and ratings were given with respect to overall mouthfeel, taste and palatability.

It was observed that the wax coated formulations showed much greater acceptance from all volunteers with respect to all the parameters, namely taste, mouth feel and overall palatability compared to uncoated tablets. Thus, coating with aqueous wax dispersion can be employed for masking the bitter taste of drug such as cetirizine hydrochloride and the coated drugs subsequently incorporated into mouth dissolve tablet Formulations.

### Example 22

### Use of aqueous wax coating dispersion for masking bitter taste of pseudoephedrine tablets

### (a) Preparation of Tablets

**Table 28 Composition of pseudoephedrine hydrochloride tablets**

| Ingredients | Mg/unit |
|---|---|
| Pseudoephedrine HCl | 60.00 |
| Dicalcium Phosphate | 95.00 |
| Povidone (Kollidone 30) | 5.00 |
| Polyethylene glycol 6000 | 20.00 |
| Aerosil 200 | 2.00 |

Dicalcium Phosphate and povidone were dry mixed and granulated with water. The wet mass was dried at 40°C for 30 min in tray drier and then mixed with Pseudoephedrine hydrochloride. The blend was lubricated and compressed into tablets.

### (b) Coating of tablets

The tablets were placed in the coating vessel of a fluidized bed processor. The tablets were sprayed with the coating emulsion of Example 2 employing methods known in the art. The inlet temperature was kept at 60°C. The spraying was carried out continuously until the desired coating level was achieved. The process was continued for coating levels of 3 % and 5%.

### (c) Evaluation of tablets with respect to perception of bitter taste

A panel of 10 healthy human volunteers was selected and all the volunteers were given one tablet each of both the samples i.e. wax coated Pseudoephedrine tablets and uncoated Pseudoephedrine tablets as control. The time required for perception of bitter taste was recorded by volunteers. For uncoated tablets, the bitter taste of drug was perceived immediately (within one minute). However for wax coated tablets, the bitterness was not perceived even after 5 minutes. Thus, aqueous wax coating dispersion can be employed for masking the bitter taste of drugs incorporated into a tablet.

## Claims

1. A aqueous wax coating emulsion comprising
a) emulsified wax wherein wax content is at 0.5 to 9% by weight and an emulsifying agent at 0.1 to 10 % by weight;
b) a plasticizer at 5 to 40 % by weight of the wax; and
c) water

2. The aqueous wax coating emulsion as claimed in claim 1 wherein the said wax is of plant, animal or synthetic origin is selected from one or more of hydrogenated vegetable oils, carnauba wax, candelilla wax, spermaceti, beeswax, montan wax, microcrystalline wax, lecithin, hydrogenated tallow, paraffin wax, shellac wax, petrolatum, compritol, cetyl alcohol, cetostearyl alcohol, precirol, stearic acid and polyethylene.

3. The aqueous wax coating emulsion as claimed in claim 1 wherein the said wax is preferably one or more of hydrogenated vegetable oils.

4. The aqueous wax coating emulsion as claimed in claim 1 wherein amount of said wax is about 2.5-7.5% by the total weight of the emulsion.

5. The aqueous wax coating emulsion as claimed in claim 1 wherein the said emulsifying agent is a non-ionic or ionic emulsifier.

6. The aqueous wax coating emulsion as claimed in claim 1 wherein emulsifying agent is selected from one or more of non ionic emulsifiers such as mono and diglycerides like glyceryl monooleate (Peceol), medium chain glycerides (capmul), glyceryl ricinoleate, glyceryl laurate, glyceryl caprylate, PEG sorbitan fatty acid esters such as PEG-20 sorbitan monolaurate (Tween 20), PEG 20 sorbitan monostearate (Tween 60), PEG sorbitan monooleate (Tween 80), Sorbitan fatty acid esters like sorbitan monolaurate (span 20), sugar ester surfactants like sucrose distearate (sucro ester 7), ionic emulsifiers such as sodium caprylate, sodium lauryl sulphate, phophlipids, alginate salts.

7. The aqueous wax coating emulsion as claimed in claim 1 wherein the preferred emulsifying agent is a medium chain glyceride.

8. The aqueous wax coating emulsion as claimed in claim 1 wherein the amount of said emulsifying agent is about 0.5 to about 7.5 percent by weight of the total weight of the emulsion.

9. The aqueous wax coating emulsion as claimed in claim 8 wherein the amount of said emulsifying agent is about 0.75 to about 5 percent by weight of the total weight of the emulsion.

10. The aqueous wax coating emulsion as claimed in claim 1 wherein the preferred plasticizer is a polymeric selected from one or more of polyvinyl alcohol, ammonio methacrylate co-polymer, Eudragit RL, Eudragit RS, polyvinylpyrrolidone, polyvinyl acetate, mixture of polyvinyl acetate with povidone (Kollidone SR™) methylcellulose, ethyl cellulose, sodium carboxy methylcellulose, hydroxypropylcellulose, hydroxypropylmethyl cellulose, polyethylene glycol, cellulose acetate, cellulose propionate (lower, medium or higher molecular weight), cellulose acetate propionate, cellulose acetate butyrate, cellulose acetate phthalate, cellulose triacetate, poly (methyl methacrylate), poly (ethyl methacrylate), poly (butyl methacrylate), poly (isobutyl methacrylate), and poly (hexyl methacrylate), poly (isodecyl methacrylate), poly (lauryl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene) high density, poly (ethylene oxide), poly (ethylene terphthalate), poly (vinyl isobutyl ether), poly (vinyl acetate), poly (vinyl chloride) or polyurethane, polymers based on acrylate and/or methacrylates).

11. The aqueous wax coating emulsion as claimed in claim 1 wherein the preferred plasticizer is selected from polyvinyl alcohol, hydroxypropylcellulose and hydroxypropylmethyl cellulose.

12. The aqueous wax coating emulsion as claimed in claim 1 wherein the amount of said plasticizer is about 7.5 to about 35 % of the concentration of the wax.

13. The aqueous wax coating emulsion as claimed in claim 12 wherein the amount of said plasticizer is about 10 to about 30% of the concentration of the wax.

14. A method of preparing an aqueous wax coating emulsion as claimed in claim1 comprising
a) heating the wax with emulsifying agent at a temperature above the melting point of the wax;
b) adding a part of hot water under stirring to the mixture of step (a);
c) cooling to room temperature to get a smooth, stable emulsion; and
d) adding the solution of plasticizer in the remaining amount of water to the mixture of step (c) under stirring.

15. A method of preparing an aqueous wax coating emulsion as claimed in claim comprising
a) heating the wax with emulsifying agent and plasticizer at a temperature above the highest melting component of the blend;
b) adding the a part of hot water under stirring to the mixture of step (a);
c) cooling to room temperature to get a smooth, stable emulsion; and
d) adding the remaining amount of water to the mixture of step (c) under stirring.

16. The use of the aqueous wax coating composition as claimed in any preceding claims as a coating agent that renders a continuous and uniform film formation.

17. The use of an aqueous wax coating emulsion as claimed in the preceeding claims for taste masking of drugs.

18. The use of an aqueous wax coating emulsion as claimed in the preceding claims for the controlled release formulations.

19. The use of the aqueous wax coating composition as claimed in claims 1 - 11 to provide a pulsatile delivery of drug from an active agent solid dosage form.

20. The use of an aqueous wax coating emulsion as claimed in the preceeding claims for stabilization of drug.

## Patentansprüche

1. Eine wässrige Wachsbeschichtungsemulsion, umfassend:
a) emulgiertes Wachs, worin der Wachsgehalt etwa 0,5 bis 9 Gew.-% und der Emulgatorgehalt etwa 0,1 bis 10 Gew.-% beträgt;
b) einen Weichmacher mit 40 Gew.-% des Wachses; und
c) Wasser

2. Die wässrige Wachsbeschichtungsemulsion, wie in Anspruch 1 beansprucht, worin das genannte Wachs pflanzlichen, tierischen oder synthetischen Ursprungs ist und ausgewählt wird aus einem oder mehreren von gehärtetem Pflanzenöl, Carnaubawachs, Candelillawachs, Walrat, Bienenwachs, Montanwachs, mikrokristallinem Wachs, Lecithin, gehärtetem Talg, Paraffinwachs, Schellackwachs, Vaseline, Compritol, Cetylakohol, Cetostearylalkohol, Precirol, Stearinsäure und Polyethylen.

3. Die wässrige Wachsbeschichtungsemulsion, wie in Anspruch 1 beansprucht, worin das genannte Wachs bevorzugt eines oder mehrere von gehärteten Pflanzenölen ist.

4. Die wässrige Wachsbeschichtungsemulsion, wie in Anspruch 1 beansprucht, worin die Menge des genannten Wachses etwa 2,5 bis 7,5 % des Gesamtgewichts der Emulsion beträgt.

5. Die wässrige Wachsbeschichtungsemulsion, wie in Anspruch 1 beansprucht, worin der genannte Emulgator ein nicht ionischer oder ionischer Emulgator ist.

6. Die wässrige Wachsbeschichtungsemulsion, wie in Anspruch 1 beansprucht, worin der Emulgator ausgewählt wird aus einem oder mehreren ionischen Emulgatoren wie Mono- und Diglyceriden, etwa Glycerylmonooleat (Peceol), mittelkettigen Glyceriden (Capmul), Glycerylricinoleat, Glyceryllaurat, Glycerylcaprylat, PEG Sorbitan-Fettsäureester wie PEG-20 Sorbitan-Monolaurat (Tween 20), PEG 20 Sorbitan-Monostearat (Tween 60), PEG-Sorbitan-Monooleat (Tween 80), SorbitanFettsäureester wie Sorbitan-Monolaurat (Span 20), Zucker-Ester-Tenside wie Saccharose-Distearat (Zuckerester 7), ionische Emulgatoren wie ein Natriumcaprylat, Natriumlaurylsulfat, Phospholipide, Alginatsalze.

7. Die wässrige Wachsbeschichtungsemulsion, wie in Anspruch 1 beansprucht, worin der bevorzugte Emulgator ein mittelkettiges Glycerid ist.

8. Die wässrige Wachsbeschichtungsemulsion, wie in Anspruch 1 beansprucht, worin die Menge des genannten Emulgators ca. 0,5 bis ca. 7,5 Gew.-% des Gesamtgewichts der Emulsion beträgt.

9. Die wässrige Wachsbeschichtungsemulsion, wie in Anspruch 8 beansprucht, worin die Menge des genannten Emulgators etwa 0,75 bis etwa 5 Gew.-% des Gesamtgewichts der Emulsion beträgt.

10. Die wässrige Wachsbeschichtungsemulsion, wie in Anspruch 1 beansprucht, worin der bevorzugte Weichmacher ein polymerer Weichmacher ist, der ausgewählt wird aus einem oder mehreren von Polyvinylalkohol, Ammoniummethacrylat-Copolymer, Eudragit RL, Eudragit RS, Polyvinylpyrrolidon, Polyvinylacetat, einer Mischung aus Polyvinylacetat mit Povidon (Kollidone SR^{™}), Methylcellulose, Ethylcellulose, Natriumcarboxymethylcellulose, Hydroxypropylcellulose, Hydroxypropylmethylcellulose, Polyethylenglycol, Celluloseacetat, Cellulosepropionat (niedrigeres, mittleres und höheres Molekulargewicht), Celluloseacetatpropionat, Celluloseacetatbutyrat, Celluloseacetatphthalat, Cellulosetriacetat, Poly(methylmethacrylat), Poly(ethylmethacrylat), Poly(butylmethacrylat), Poly(isobutylmethacrylat) und Poly(hexylmethacrylat), Poly(isodecylmethacrylat), Poly(laurylmethacrylat), Poly(phenylmethacrylat), Poly(methylacrylat), Poly(isopropylacrylat), Poly(isobutylacrylat), Poly(octadecylacrylat), Poly(ethylen), Poly(ethylen) geringer Dichte, Poly(ethylen) hoher Dichte, Poly(ethylenoxid), Poly(ethylenterphthalat), Poly(vinylisobutylether), Poly(vinylacetat), Poly(vinylchlorid) oder Polyurethan, Acrylat- und/oder Methacrylat-basierte Polymere).

11. Die wässrige Wachsbeschichtungsemulsion, wie in Anspruch 1 beansprucht, worin der bevorzugte Weichmacher ausgewählt wird aus Polyvinylalkohol, Hydroxypropylcellulose und Hydroxypropylmethylcellulose.

12. Die wässrige Wachsbeschichtungsemulsion, wie in Anspruch 1 beansprucht, worin die Menge des genannten Weichmachers etwa 7,5 bis etwa 35% der Wachskonzentration beträgt.

13. Die wässrige Wachsbeschichtungsemulsion, wie in Anspruch 12 beansprucht, worin die Menge des genannten Weichmachers etwa 10 bis etwa 30% der Wachskonzentration beträgt.

14. Ein Verfahren zur Herstellung einer wässrigen Wachsbeschichtungsemulsion, wie in Anspruch 1 beansprucht, umfassend:
a) Erhitzen des Wachses mit einem Emulgator bei einer Temperatur über dem Schmelzpunkt des Wachses;
b) Hinzufügen von einem Teil heißes Wasser unter Rühren zur Mischung von Schritt (a);
c) Abkühlen auf Raumtemperatur, um eine glatte, stabile Emulsion zu erhalten; und
d) Hinzufügen der Weichmacherlösung in der restlichen Menge Wasser unter Rühren zur Mischung von Schritt (c).

15. Ein Verfahren zur Herstellung einer wässrigen Wachsbeschichtungsemulsion, wie in Anspruch 1 beansprucht, umfassend:
a) Erhitzen des Wachses mit einem Emulgator und Weichmacher bei einer Temperatur über der höchsten Schmelzkomponente der Mischung;
b) Hinzufügen von einem Teil heißes Wasser unter Rühren zur Mischung von Schritt (a);
c) Abkühlen auf Raumtemperatur, um eine glatte, stabile Emulsion zu erhalten; und
d) Hinzufügen der restlichen Menge Wasser unter Rühren zur Mischung von Schritt (c).

16. Die Verwendung der wässrigen Wachsbeschichtungszusammensetzung, wie in irgendeinem der vorhergehenden Ansprüche beansprucht, als ein Beschichtungsmittel, das eine kontinuierliche und einheitliche Filmbildung ergibt.

17. Die Verwendung einer wässrigen Wachsbeschichtungsemulsion, wie in den vorhergehenden Ansprüchen beansprucht, zur Geschmacksmaskierung von Arzneimitteln.

18. Die Verwendung einer wässrigen Wachsbeschichtungsemulsion, wie in den vorhergehenden Ansprüchen beansprucht, für die Formulierungen mit kontrollierter Wirkstofffreigabe.

19. Die Verwendung der wässrigen Wachsbeschichtungsemulsion, wie in den Ansprüchen 1 bis 11 beansprucht, um eine pulsatile Abgabe des Arzneimittels aus einer festen Darreichungsform eines Wirkstoffs bereitzustellen.

20. Die Verwendung einer wässrigen Wachsbeschichtungsemulsion, wie in den vorhergehenden Ansprüchen beansprucht, zur Stabilisierung eines Arzneimittels.

## Revendications

1. Une émulsion de revêtement de cire aqueuse contenant
a) une cire émulsifiée dans laquelle la teneur en cire est 0,5 à 9% en poids et un agent émulsifiant de 0,1 à 10% en poids,
b) un plastifiant de 5 à 40% en poids de la cire, et
c) de l'eau.

2. L'émulsion de revêtement de cire aqueuse selon la Revendication 1 où ladite cire est d'origine végétale, animale ou synthétique et est sélectionnée parmi un ou plusieurs produits parmi huiles végétales hydrogénées, cire de carnauba, cire de candelilla, blanc de baleine, cire d'abeille, cire de montan, cire microcristalline, lécithine, suif hydrogéné, cire de paraffine, cire de gommelaque, pétrolatum, compritol, alcool cétylique, alcool cétostéaryle, precirol, acide stéarique et polyéthylène.

3. L'émulsion de revêtement de cire aqueuse selon la Revendication 1 où ladite cire est de préférence une ou plusieurs huiles végétales hydrogénées.

4. L'émulsion de revêtement de cire aqueuse selon la Revendication 1 où une quantité de ladite cire est d'environ 2,5 à 7,5% en poids total de l'émulsion.

5. L'émulsion de revêtement de cire aqueuse selon la Revendication 1 où ledit agent émulsifiant est un émulsifiant ionique ou non ionique.

6. L'émulsion de revêtement de cire aqueuse selon la Revendication 1 où l'agent émulsifiant est sélectionné parmi un ou plusieurs émulsifiants non ioniques tels que mono et diglycérides tels que monooléate de glycéryle (Peceol), glycérides à chaîne moyenne (capmul), ricinoléate de glycéryle, laurate de glycéryle, caprylate de glycéryle, esters d'acide gras de sorbitane PEG tels que monolaurate de sorbitane PEG-20 (Tween 20), monostéarate de sorbitane PEG 20 (Tween 60), monooléate de sorbitane PEG (Tween 80), esters d'acide gras de sorbitane tels que monolaurate de sorbitane (span 20), tensioactifs d'ester de sucre tels que distéarate de sucrose (sucroester 7), émulsifiants ioniques tels que caprylate de sodium, sulfate de lauryle de sodium, phospholipides, sels d'alginate.

7. L'émulsion de revêtement de cire aqueuse selon la Revendication 1 où l'agent émulsifiant préféré est un glycéride à chaîne moyenne.

8. L'émulsion de revêtement de cire aqueuse selon la Revendication 1 où la quantité dudit agent émulsifiant est d'environ 0,5 à environ 7,5% en poids du poids total de l'émulsion.

9. L'émulsion de revêtement de cire aqueuse selon la Revendication 8 où la quantité dudit agent émulsifiant est d'environ 0,75 à environ 5% en poids du poids total de l'émulsion.

10. L'émulsion de revêtement de cire aqueuse selon la Revendication 1 où le plastifiant préféré est un polymère sélectionné parmi un ou plusieurs produits parmi alcool polyvinylique, copolymère ammonio méthacrylate, Eudragit RL, Eudragit RS, polyvinylepyrrolidone, acétate de polyvinyle, mélange d'acétate de polyvinyle avec povidone (Kollidone SR^{™}) méthylecellulose, cellulose d'éthyle, sodium carboxy méthylecellulose, hydroxypropylecellulose, hydroxypropyleméthylecellulose. polyéthylène glycol, acétate de cellulose, propionate de cellulose (poids moléculaire inférieur, moyen ou supérieur), propionate d'acétate de cellulose, butyrate d'acétate de cellulose, phtalate d'acétate de cellulose, triacétate de cellulose, poly (méthyle méthacrylate), poly (éthyle méthacrylate), poly (butyle méthacrylate), poly (isobutyle méthacrylate), et poly (hexyle méthacrylate), poly (isodécyle méthacrylate), poly (lauryle méthacrylate), poly (phényle méthacrylate), poly (méthyle acrylate), poly (isopropyle acrylate), poly (isobutyle acrylate), poly (octadécyle acrylate), poly (éthylène), poly (éthylène) faible densité, poly (éthylène) haute densité, poly (oxyde d'éthylène), poly (téréphtalate d'éthylène), poly (éther isobutylique de vinyle), poly (acétate de vinyle), poly (chlorure de vinyle) ou polyuréthane, polymères basés sur acrylate et/ou méthacrylate).

11. L'émulsion de revêtement de cire aqueuse selon la Revendication 1 où le plastifiant préféré est sélectionné parmi alcool de polyvinyle, hydroxypropylecellulose et hydroxypropyleméthylecellulose.

12. L'émulsion de revêtement de cire aqueuse selon la Revendication 1 où la quantité dudit plastifiant est d'environ 7,5 à environ 35% de la concentration de la cire.

13. L'émulsion de revêtement de cire aqueuse selon la Revendication 12 où la quantité dudit plastifiant est d'environ 10 à environ 30% de la concentration de la cire.

14. Un procédé de préparation d'une émulsion de revêtement de cire aqueuse selon la Revendication 1 comprenant
a) le chauffage de la cire avec un agent émulsifiant à une température supérieure au point de fusion de la cire,
b) l'ajout d'une partie d'eau chaude sous agitation au mélange de l'opération (a),
c) le refroidissement à température ambiante de façon à obtenir une émulsion stable et fluide, et
d) l'ajout de la solution de plastifiant dans la quantité restante d'eau au mélange de l'opération (c) sous agitation.

15. Un procédé de préparation d'une émulsion de revêtement de cire aqueuse selon la Revendication 1 comprenant
a) le chauffage de la cire avec un agent émulsifiant et un plastifiant à une température supérieure à celle du composant présentant le point de fusion le plus élevé du mélange,
b) l'ajout de la partie d'eau chaude sous agitation au mélange de l'opération (a),
c) le refroidissement à température ambiante de façon à obtenir une émulsion stable et fluide, et
d) l'ajout de la quantité restante d'eau au mélange de l'opération (c) sous agitation.

16. L'utilisation de la composition de revêtement de cire aqueuse selon l'une quelconque des Revendications précédentes en tant qu'agent de revêtement qui rend une formulation de film continue et uniforme.

17. L'utilisation d'une émulsion de revêtement de cire aqueuse selon l'une quelconque des Revendications précédentes pour le masquage du goût de médicaments.

18. L'utilisation d'une émulsion de revêtement de cire aqueuse selon l'une quelconque des Revendications précédentes pour des formulations à libération contrôlée.

19. L'utilisation de la composition de revêtement de cire aqueuse selon l'une quelconque des Revendications 1 à 11 de façon à fournir une administration pulsatile d'un médicament à partir d'une forme posologique solide d'un agent actif.

20. L'utilisation d'une émulsion de revêtement de cire aqueuse selon l'une quelconque des Revendications précédentes pour la stabilisation d'un médicament.
